# EUROPÄISCHE PATENTSCHRIFT

(11) **EP 2 196 199 B1**
(45) Veröffentlichungstag und Bekanntmachung des Hinweises auf die Patenterteilung: **13.08.2014**
(21) Anmeldenummer: 10158404.3
(22) Anmeldetag: 19.01.2006
(51) Int. Cl.: A61K 31/00, A61K 31/5513, A61K 31/195, A61K 31/4422, A61P 25/00, A61P 27/16

(54) **Behandlung von Phantomphänomenen**
Phantom phenomena treatment
Traitement de phenomenes fantomes

(30) Priorität: 25.01.2005 DE 102005004343
(43) Veröffentlichungstag der Anmeldung: 16.06.2010
(62) Teilanmeldung aus: 06706302.4
(73) Patentinhaber: Auris Medical AG, 4001 Basel (CH)
(72) Erfinder: Knipper, Dr. Marlies, 70771, Leinfelden-Echterdingen Stetten (DE); Rüttiger, Dr. Lukas, 72127, Kusterdingen (DE)
(74) Vertreter: Graf von Stosch, Andreas

(56) Entgegenhaltungen:
- WO-A-2005/073237
- DE-A1- 10 124 953
- THEOPOLD H M: "[Nimodipine (Bay e 9736) a new therapy concept in diseases of the inner ear?]" LARYNGOLOGIE, RHINOLOGIE, OTOLOGIE. DEC 1985, Bd. 64, Nr. 12, Dezember 1985 (1985-12), Seiten 609-613, XP009065317 ISSN: 0340-1588
- CIOCON J O ET AL: "Does oxazepam offer relief of tinnitus or alter it to a non-troublesome functional level in the elderly?" JOURNAL OF THE AMERICAN GERIATRICS SOCIETY, Bd. 45, Nr. 9, 1997, Seite S41, XP009065300 & ANNUAL MEETING OF THE AMERICAN GERIATRICS SOCIETY AND AMERICAN FEDERATION FOR AGING RESEARCH ISSN: 0002-8614
- MENKES DAVID B ET AL: "Sodium valproate for tinnitus" JOURNAL OF NEUROLOGY NEUROSURGERY AND PSYCHIATRY, Bd. 65, Nr. 5, November 1998 (1998-11), Seite 803, XP002240622 ISSN: 0022-3050
- SZCZEPANIAK WILLIAM S ET AL: "Effect of L-baclofen and D-baclofen on the auditory system: A study of click-evoked potentials from the inferior colliculus in the rat" ANNALS OF OTOLOGY RHINOLOGY AND LARYNGOLOGY, Bd. 104, Nr. 5, 1995, Seiten 399-404, XP009065327 ISSN: 0003-4894
- WEBER W E: "[Pharmacotherapy for neuropathic pain caused by injury to the afferent nerve fibers]" NEDERLANDS TIJDSCHRIFT VOOR GENEESKUNDE. 28 APR 2001, Bd. 145, Nr. 17, 28. April 2001 (2001-04-28), Seiten 813-817, XP002377166 ISSN: 0028-2162
- VICHITRANANDA C ET AL: "Midazolam for the treatment of phantom limb pain exacerbation: preliminary reports." JOURNAL OF THE MEDICAL ASSOCIATION OF THAILAND = CHOTMAIHET THANGPHAET. FEB 2001, Bd. 84, Nr. 2, Februar 2001 (2001-02), Seiten 299-302, XP9065335 ISSN: 0125-2208
- EGGERMONT JOS J ET AL: "The neuroscience of tinnitus." TRENDS IN NEUROSCIENCES. NOV 2004, Bd. 27, Nr. 11, November 2004 (2004-11), Seiten 676-682, XP004595923 ISSN: 0166-2236
- GUITTON M J ET AL: "SALICYLATE INDUCES TINNITUS THROUGH ACTIVATION OF COCHLEAR NMDA RECEPTORS" JOURNAL OF NEUROSCIENCE, NEW YORK, NY, US, Bd. 23, Nr. 9, 1. Mai 2003 (2003-05-01), Seiten 3944-3952, XP008054642 ISSN: 0270-6474
- SIFRINGER M ET AL: "Neurotrophin-downregulation in the developing brain following treatment with NMDA-antagonists" ABSTRACTS OF THE ANNUAL MEETING OF THE SOCIETY FOR NEUROSCIENCE, SOCIETY FOR NEUROSCIENCE, WASHINGTON, DC, US, Bd. 27, Nr. 2, 1. Januar 2001 (2001-01-01) , Seite 1739, XP009133107 ISSN: 0190-5295
- JIANG X ET AL: "CRE - binding protein and NF - KAPPA B are required for N - methyl - D aspartate - mediated BDNF promoter III activity in rat hippocampal neurons" ABSTRACTS OF THE ANNUAL MEETING OF THE SOCIETY FOR NEUROSCIENCE, SOCIETY FOR NEUROSCIENCE, WASHINGTON, DC, US, Bd. 33, 1. Januar 2003 (2003-01-01), XP009133108 ISSN: 0190-5295
- GUITTON M J ET AL: "SALICYLATE INDUCES TINNITUS THROUGH ACTIVATION OF COCHLEAR NMDA RECEPTORS", JOURNAL OF NEUROSCIENCE, NEW YORK, NY, US, vol. 23, no. 9, 1 May 2003 (2003-05-01), pages 3944-3952, XP008054642, ISSN: 0270-6474
- FUKUSHIMA KOUJI ET AL: "Inhibitory effects of MK801 on activation of MAP kinase in adult rat hippocampus after intracerebroventricular injections of glutamate, somatostatin and neuropeptide Y", JAPANESE JOURNAL OF PHARMACOLOGY, vol. 79, no. SUPPL. 1, 1998, page 65P, XP009158312, & 71ST ANNUAL MEETING OF THE JAPANESE PHARMACOLOGICAL SOCIETY; KYOTO, JAPAN; MARCH 23-26, 1998 ISSN: 0021-5198

## Beschreibung

Die vorliegende Erfindung betrifft eine Substanz zur Behandlung von Phantomphänomenen, nämlich dem akuten Tinnitus.

Derartige Substanzen sind im Stand der Technik allgemein bekannt.

Unter dem Phantomphänomen des Tinnitus werden von einem Patienten wahrgenommene Geräusche verstanden, die durch das Ohr und das auditorische System erzeugt werden. Tinnitus, der erst seit wenigen Wochen bis drei Monaten besteht, wird als akuter Tinnitus bezeichnet. Besteht der Tinnitus länger als ein Jahr, wird er als chronisch bezeichnet. Nach epidemiologischen Erhebungen sind in Deutschland ca. drei Millionen Erwachsene, d.h. etwa 4 % der Bevölkerung, von chronischem Tinnitus betroffen. Global betrachtet kommt es jährlich bei ca. zehn Millionen Menschen zu Tinnitus, der bei ca. 340.000 von einer akuten in eine chronische Form übergeht, sog. Neuerkrankungsrate.

Zu den vielfältigen Ursachen des Tinnitus gehören chronische Lärmschädigungen, akute Knallverletzungen des Gehörs, Hörsturz und anderweitige Erkrankungen, die mit einem Hörverlust einhergehen. Zusammenhänge mit Innenohrschwerhörigkeit als chronischfortschreitende Form oder als Lärmschwerhörigkeit, gefolgt von Morbus Menière und Hörsturz stehen gemäß klinischen Studien bei mehr als zwei Drittel im Zusammenhang mit Tinnitus. An der Entstehung und Aufrechterhaltung des Tinnitus sind auch Erkrankungen der Halswirbelsäule sowie des Kiefergelenks und Kaumuskelsystems beteiligt. Tinnitus scheint auch eine psychische Komponente aufzuweisen, so dass in diesem Zusammenhang von psychogenem Tinnitus gesprochen wird. In vielen Fällen lässt sich jedoch trotz intensiver Diagnostik keine sichere Ursache des Tinnitus erkennen.

Derzeit wird Tinnitus mit psychosomatischer Behandlung, Relaxationstherapie, Biofeedback, Hypnotherapie, elektrischer Stimulation, Lidocain, Iontophorese oder Maskierung therapiert. Hierbei handelt es sich jedoch um ausschließlich symptomatische Therapiekonzepte.

Die WO 02/15907 A1 schlägt die Behandlung von Tinnitus durch die Verabreichung des Kalium-Kanal-Öffners Flupirtin vor. Diese Behandlung hat den Nachteil, dass es sich bei Flupirtin zusätzlich um ein muskelrelaxierendes Analgetikum handelt, wodurch eine Applikation mit nicht zu tolerierenden Nebenwirkungen verbunden wäre.

Wang et al. (2000), Evaluating effects of some medicine on tinnitus with animal behavioral model in rats, Zhonghua Er. Bi. Yan. Hou. Ke. Za. Zhi. 35 (5), abstract, schlagen die Verabreichung von Nimodipin zur Behandlung von Tinnitus vor. Bei Nimodipin handelt es sich um einen Inhibitor des Ca⁺⁺-Kanals Cav1.3. Dabei hat sich allerdings ergeben, dass die Blockade des Cav1.3-Kanals im auditorischen System unmittelbar zur Taubheit führen würde, so dass Nimodipin für die Behandlung von Tinnitus gänzlich ungeeignet ist.

In der WO 2004/022069 A1 werden als eine der möglichen Ursachen von Tinnitus aberrante NMDA(N-Methyl-D-Aspartat)-Rezeptoren beschrieben. Diese veränderten sog. Glutamatrezeptorkanäle, die u.a. von auditorischen Nervenzellen exprimiert werden, führen zu verstärktem Einstrom von Calcium in die Zelle. In diesem Dokument wird vorgeschlagen, zur Behandlung von Tinnitus NMDA-Rezeptor-Antagonisten einzusetzen. Völlig unklar bleibt allerdings, ob mit diesen Substanzen die akute oder chronische Situation des Tinnitus zu behandeln ist. Ferner finden sich keinerlei Hinweise, wie die Applikation der Substanzen zu erfolgen hat.

In der DE 101 24 953 A1 wird ein Behandlungskonzept bei Tinnitus vorgeschlagen, das in der Stimulation der Expression des "Brain-derived Nerve Growth Factors" (BDNF) liegt. Die dortigen Autoren beschreiben auf der Grundlage eines Tiermodelles, dass bei chronischem Tinnitus in der Cochlea und im inferioren Colliculus eine Erniedrigung der BDNF-Expression vorherrscht, weshalb als therapeutischer Ansatz die Stimulation der BDNF-Expression vorgeschlagen wird. Die dortigen Autoren haben jedoch ganz konkret und ausschließlich die Situation bei chronischem Tinnitus untersucht. So wurden die in dem Tiermodell verwendeten Ratten über drei Monate mit Salicylaten behandelt, wodurch bekanntermaßen chronischer Tinnitus induziert wird; vgl. Penner M. J., und Jastreboff P. J. (1996), Tinnitus: Psycho-physical observations in humans and animal models, in: Van de Water, Popper A. N., Fax, R. R. (Ed.), Clinical aspects of hearing, Springer, New York, Heidelberg, Seiten 208 bis 304; und Bauer, C. A., et al. (1999), A behavioral model of chronic Tinnitus in rats. Otolaryngol. Head Neck Surg. 121, Seiten 457 bis 462.

Nicht erkannt wurde von den Autoren der DE 101 24 953 A1 jedoch, dass es signifikante Unterschiede zwischen der Behandlung von chronischem und akutem Tinnitus geben muss.

Ein Überblick über das Krankheitsbild des Tinnitus findet sich bspw. in Waddell, A., Canter, R. (2004), Tinnitus, Am. Fam. Physician 69, Seiten 591 bis 592.

Zahlreiche Veröffentlichungen beschäftigen sich mit diversen Substanzen, die potentiell zur Behandlung von Tinnitus eingesetzt werden könnten.

In WO 2005/073237 wird die Verwendung des Wirkstoffs Gaboxadol Monohydrat für die Behandlung von neurologischen und psychiatrischen Krankheiten, wie unter anderem Tinnitus, offenbart.

Theopold (Laryng. Rhinol. Otol. 64 (1985) 609-613) beschreibt die Verwendung von Nimodipin als potentielles Therapiekonzept bei Innenohrerkrankungen. Bei Nimodipin handelt es sich um ein kalziumantagonistisches Derivat der Dihydropyridine mit prädilektiver Wirkung auf das zerebrale Gefäßsystem.

Die Veröffentlichung von Menkes et al. (J. Neurol. Neurosurg. Psychiatry 1998, 65:803) beschreibt die Verwendung von Natriumvalproat für die Behandlung von Tinnitus.

Szczepaniak et al. (Ann Otol. Rhinol. Laryngol. 104:1996, Seiten 399-404) offenbart die Verwendung von Baclofen bei der Behandlung von Tinnitus im Rattenmodell.

Die Verwendung von GABA-Rezeptoragonisten zur Behandlung von Tinnitus wird von Eggermont et al. (Trends in Neurosciences, Vol. 27, Nr. 11, 2004) beschrieben.

Guitton et al. (The Journal of Neuroscience, 2003, 23(9):3944-3952) beschäftigt sich mit der Verwendung von NMDA-Antagonisten zur Behandlung von Salicylat-induziertem Tinnitus. Insbesondere kommt der NMDA-Rezeptorantagonist MK-801 in dieser Untersuchung zum Einsatz.

Schließlich beschreibt Ciocon et al. (Journal of the American Geriatric Society, 45, Nr. 9, 1997, Seite 41), dass Oxazepam zur Behandlung von Tinnitus eingesetzt werden könnte.

Aufgabe der vorliegenden Erfindung ist es deshalb, eine Substanz zur Therapie des akuten Tinnitus bereitzustellen, mit der bzw. dem die Nachteile aus dem Stand der Technik verhindert werden. Insbesondere soll eine solche Substanz bereitgestellt werden, mit der gezielt die Phantomphänomene des akuten Tinnitus behandelt werden können.

Diese Aufgabe wird durch die Bereitstellung einer mit der BDNF-Signaltransduktionskaskade interagierenden Substanz gelöst.

Bei BDNF ("Brain-derived Nerve Growth Factor") handelt es sich um ein von Neuronen des Zentralnervensystems gebildetes basisches Protein, das aus 252 Aminosäuren besteht. BDNF ist ein Wachstumsfaktor, der in die Entwicklung des Nervensystems involviert ist und u.a. eine Rolle bei der Ausbildung der Plastizität der Synapsen spielt. Die Wirkung von BDNF wird über spezielle Rezeptoren vermittelt, bspw. über den BDNF-Rezeptor trkB, der wiederum nachgeschaltete Faktoren, wie die MAP-Kinase oder Cam-Kinase in ihrer Aktivität bzw. Wirkungsweise reguliert. BDNF wird wiederum selbst reguliert, bspw. über Calcium.

Sämtliche Faktoren, die BDNF in seiner Aktivität, Expression, Wirkungsweise o.Ä. beeinflussen oder regulieren, bzw. sämtliche Faktoren, die durch BDNF auf diese Art und Weise beeinflusst oder reguliert werden, bilden die sog. BDNF-Signaltransduktionskaskade.

Die BDNF-Signaltransduktionskaskade kann unterteilt werden in eine Kaskade, die dem BDNF-Rezeptor trkB vorgeschaltet, und eine, die diesem nachgeschaltet ist. Die trkB-nachgeschaltete Signaltransduktionskaskade wird durch die Bindung von BDNF sowie anderer Mitglieder der Neurotropinfamilie an den trk-Rezeptor eingeleitet. Dies führt zu einer trk-Dimerisierung und Aktivierung der Tyrosinkinaseaktivität des Rezeptors. Durch diese ligandenvermittelte Aggregation der Rezeptoren kommt es zur Autophosphorylierung intrazellulärer Domänen, die eine Aktivierung von Signalmolekülen, wie der Phospholipase C (PLC), der Phosphatidyl-Inositol-3-Kinase (PI3-Kinase) und des Adapterproteins Shc (SH-2-enthaltendes Protein), zur Folge haben. Die weiteren Signale, vor allem vermittelt über die ras-abhängige MAP-Kinase, beeinflusst letztendlich zelluläre Gentranskription und Proteinsynthese.

Die trkB-vorgeschaltete Kaskade betrifft die Regulation von BDNF. So ist es bekannt, dass die BDNF-Expression aktivitätsabhängig durch unterschiedliche Stimuli reguliert wird, wie elektrische Stimulation oder Verletzung, reine physische Bewegung oder auch durch den Tagesrhythmus. Vorstehend genannte Stimuli regulieren die Expression verschiedener nicht-translatierter BDNF-Exone, die dann zusammen mit dem gemeinsamen 5'-Exon letztlich unterschiedliche BDNF-Transkripte bilden. Die Stimuli wirken offenbar über unterschiedliche Ca⁺⁺-induzierte Signalkaskaden auf die Promotoren der verschiedenen BDNF-Exone.

Erfindungsgemäß wird die Blockade oder Inhibition des BDNF-Rezeptors und die diesem Rezeptor nachgeschaltete BDNF-Signaltransduktionskaskade adressiert. Es versteht sich, dass auch trkB selbst Bestandteil der BDNF-Signaltransduktionskaskade ist.

Ein Überblick über die BDNF-Signaltransduktionskaskade findet sich in Gabellini, N. (2004), Transcriptional regulation by cAMP and Ca++ links the Na+/Ca++ exchanger 3 to memory and sensory pathways, Mol. Neurobiol. 30, Seiten 91 bis 116; West et al. (2001), Calcium Regulation of Neuronal Gene Expression, Proc. Natl. Acad. Sci. USA 98, Seiten 11024-11031. Die Inhalte dieser Publikationen sind durch Inbezugnahme Bestandteile der vorliegenden Anmeldung.

BDNF spielt bekanntermaßen bei einer Vielzahl von Krankheiten eine Rolle, vgl. Binder, D. K. (2004), The role of BDNF in epilepsy and other diseases of the mature nervous system, Recent Advances in Epilepsy Research, Seiten 34 bis 56.

Erfindungsgemäß kann sich eine die Blockade oder Inhibition des BDNF-Rezeptors bewirkende oder der nachgeschalteten BDNF-Signaltransduktionskaskade interagierende Substanz in jeglicher Ausgestaltung darstellen, d.h. es kann sich um eine solche Substanz handeln, die sowohl chemisch als auch biochemisch oder biologisch definiert ist, die also eine chemisch synthetisierte Verbindung jeglicher Ausprägung ist, ein Molekül, Ion, Atom, ein Protein, Peptid, Antikörper, eine Nucleinsäure, ein Aptamer, ein Virus, Bakterium etc. darstellt.

Unter Interaktion wird erfindungsgemäß die direkte oder indirekte Wechselwirkung dieser Substanz mit einem Faktor der nachgeschalteten BDNF-Signaltransduktionskaskade verstanden, die in einer Modifizierung der physiologischen Signaltransduktion innerhalb dieser Kaskade resultiert. Derartige interagierende Substanzen sind im Stand der Technik hinreichend beschrieben.

Die Aufgabe der vorliegenden Erfindung wird mit der Bereitstellung einer solchen Substanz vollständig gelöst. Insbesondere haben die Erfinder erstmals eine Verwendung derartiger Substanzen zur Therapie für die Phantomphänomene des akuten Tinnitus bereitgestellt.

Es kann nun dank der Erfindung gezielt die akute Ausprägung des Tinnitus behandelt werden und somit im Vorfeld die problematische Chronifizierung dieses Phantomphänomens verhindert werden. Im Stand der Technik wird häufig keinerlei Differenzierung zwischen chronischem und akutem Tinnitus vorgenommen und vielfach lediglich Ansätze zur Behandlung von chronischem Tinnitus vorgeschlagen, wie auch in der oben erwähnten DE 101 24 953 A1.

Erfindungsgemäß ist es dabei bevorzugt, wenn eine solche interagierende Substanz vorgesehen wird, die eine Blockade oder Inhibition des BDNF-Rezeptors oder der diesem Rezeptor nachgeschalteten BDNF-Signaltransduktionskaskade bewirkt.

Erfindungsgemäß wird in diesem Sinne unter einer Blockade oder Inhibition verstanden, dass die Signalweiterleitung innerhalb der BDNF-Signaltransduktionskaskade gegenüber der physiologischen Situation verlangsamt, verschlechtert, reduziert oder völlig unterdrückt wird.

Diese Erkenntnis der Erfinder war völlig überraschend und steht im Gegensatz zu Erkenntnissen, die in der DE 101 24 953 A1 für chronischen Tinnitus beschrieben sind. So wird nämlich in dem vorstehend genannten Dokument eine Stimulation der BDNF-Expression gefordert, wohingegen gemäß der Erfindung zur Behandlung von Phantomphänomenen, wie dem akuten Tinnitus, eine Inhibition der BDNF-Expression bzw. BDNF-Signaltransduktion gefordert wird. Die Erfinder haben deshalb erkannt, dass bei akutem Tinnitus eine Therapie zu erfolgen hat, die genau entgegengesetzt ausgestaltet ist, wie dies gemäß dem vorgenannten Dokument für chronischen Tinnitus vorgeschlagen wird. Eine Übertragung dieses bekannten Konzeptes auf den akuten Zustand hätte deshalb fatale Folgen.

Die Erfinder der vorliegenden Erfindung haben bei Ratten durch kurzzeitige Gabe von Salicylaten akuten Tinnitus induziert und in den cochleären Ganglien eine verstärkte Expression von BDNF festgestellt. Die Erfinder konnten nachweisen, dass die Verhältnisse bei der Induktion von chronischem Tinnitus genau umgekehrt liegen, es dort also zu einer verminderten Expression von BDNF in den cochleären Ganglien kommt.

Es hat sich also überraschenderweise gezeigt, dass eine Differenzierung zwischen akutem und chronischem Tinnitus für die Wahl der richtigen Behandlungsmethode essentiell ist und bislang nicht oder nicht ausreichend im Stand der Technik erfolgt ist. Akuter Tinnitus ist deshalb erfindungsgemäß über eine Inhibition bzw. Blockade der BDNF-Signaltransduktionskaskade, bspw. durch Hemmung der BDNF-Expression, zu behandeln, wohingegen gemäß der DE 101 24 953 A1 bei chronischem Tinnitus eine Stimulation der BDNF-Expression zu erfolgen hat.

Die Erfinder präsentieren hier erstmals molekulare Daten, die die Mechanismen der Pathologie von Tinnitus aufzeigen, und bereichern damit die Grundlagenwissenschaften und die Medizin um ein besseres Verständnis über diese Phantomphänomene und zeigen gleichzeitig einen ursächlichen Therapieansatz auf.

Erfindungsgemäß wird auch eine solche Substanz zur Verwendung vorgesehen, die eine Blockade oder Inhibition der trkB nachgeschalteten Signaltransduktionskaskade bewirkt.

Substanzen, die Faktoren der BDNF-Signaltransduktionskaskade inhibieren, lassen sich leicht über Aktivitäts- bzw. Inhibitionstests ermitteln, die zum Routinehandwerk eines Molekularbiologen oder eines klinischen Chemikers bzw. Pharmakologen gehören.

Erfindungsgemäß wird alternativ eine solche Substanz vorgesehen, die eine Blockade oder Inhibition des BDNF-Rezeptors (trkB) oder der diesem nachgeschalteten Signaltransduktionskaskade bewirkt.

Diese Maßnahme hat den Vorteil, dass ein weiterer geeigneter Angriffspunkt für einen therapeutischen Eingriff ausgenutzt wird. So kann trkB selbst mittels geeigneter im Stand der Technik bekannter Substanzen und damit die gesamte nachfolgende BDNF-Signaltransduktionskaskade blockiert werden. Ferner können mittels des "molecular drug designs" anhand von kristallographischen Daten über trkB geeignete, mit diesem Rezeptor interagierende Substanzen entwickelt werden, die vorteilhafte Wirkstoffe für ein Arzneimittel zur Behandlung von akutem Tinnitus oder Phantomschmerz darstellen. Auch Faktoren, die trkB in der Signaltransduktionskaskade nachgeschaltet sind, wie bspw. PLC, PI3-Kinase oder Shc, sind geeignete Angriffspunkte für eine erfindungsgemäße Verwendung der Substanz. Eine Stilllegung oder Inhibition dieser Faktoren führt ebenfalls zu einer geeigneten Blockade der BDNF-Signaltransduktionskaskade.

Dabei ist es bevorzugt, wenn als Substanz ein MAP-Kinase-, ein Cam-Kinase-Inhibitor oder ein trkB-Antagonist vorgesehen wird. Besonders geeignete MAP-Kinase-Inhibitoren sind erfindungsgemäß die Substanzen U 0126 oder PD 98058.

Wie die Erfinder feststellen konnten, sind derartige Substanzen zur Behandlung von akutem Tinnitus besonders gut geeignet. U 0126, MEK1/2-Inhibitor, und PD 98058, ein MEK1-Inhibitor können von Cell Signalling Technology, Inc., Beverly, MA, Vereinigte Staaten von Amerika, bezogen werden. Dabei obliegt die Wahl der eingesetzten Konzentration dem Fachmann und ist abhängig von der Ausprägung der Krankheit, dem übrigen Therapiekonzept sowie verschiedenen vom zu behandelnden Patienten individuellen Faktoren. Vor diesem Hintergrund wird die eingesetzte Konzentration für den jeweiligen Einzelfall vom Fachmann unter Verwendung von Routinemaßnahmen festgelegt.

Nach einer vorteilhaften Weiterbildung der erfindungsgemäßen Verwendung wird die Substanz lokal am oder im Ohr.

Diese Maßnahme hat den Vorteil, dass die Substanz gezielt am Wirkort verabreicht wird, so dass nur geringe Mengen an Wirksubstanz erforderlich sind. Dadurch wird der Körper des behandelten Patienten im Übrigen weniger belastet und Nebenwirkungen werden weit gehend reduziert. Im Falle einer Behandlung von akutem Tinnitus bietet sich das Mikrodosiersystem an, das von Lehner, R. et al. (1996), A new implantable drug delivery system for local therapy of the middle and inner ear, Ear. Nose Throat 76, Seiten 567 bis 570, beschrieben wird.

Die lokale Applikation der Substanz kann alternativ auch über die Anwendung von bioabbaubarem Hydrogel erfolgen, das als Trägermatrix für die Substanz dient. Derartiges bioabbaubares Hydrogel wurde bereits erfolgreich im Tiermodell zur lokalen Applikation von BDNF an das runde Fenster des Innenohres angewandt; Ito et al. (2005), A new method for drug application to the inner ear, J. Otorhinolaryngol. Relat. Spec., Seiten 272-275.

Die Erfinder haben herausgefunden, dass eine lokale Applikation der mit der die Blockade oder Inhibition des BDNF-Rezeptors bewirkenden Substanz oder der mit der nachgeschalteten BDNF-Signaltransduktionskaskade interagierenden Substanz besonders vorteilhaft ist, da nämlich, wie die Erfinder in weiteren Experimenten feststellen konnten, bei akutem Tinnitus die BDNF-Expression im auditorischen Cortex im Gegensatz zur Situation in den cochleären Ganglien überraschenderweise reduziert ist. Eine systemische Applikation von die BDNF-Signaltransduktionskaskade inhibierenden Substanzen würde deshalb im Cortex zu einer noch weiteren Erniedrigung der BDNF-Expression führen und damit schädliche Effekte auf den Organismus haben. Bei akutem Tinnitus wäre deshalb eine systemische Gabe der Substanz, die die BDNF-Signaltransduktionskaskade inhibiert, kontraindikativ.

Ein weiterer Gegenstand der vorliegenden Erfindung betrifft eine Substanz zur therapeutischen und/oder prophylaktischen Behandlung der Phantomphänomene des akuten Tinnitus bei einem menschlichen oder tierischen Lebewesen, die ausgewählt ist aus der Gruppe bestehend aus: MAP-Kinase-Inhibitor, insbesondere U 0126 oder PD 98058, Cam-Kinase-Inhibitor und trkB-Antagonist.

Die Erfinder haben erstmals eine konkrete medizinische Anwendung der vorgenannten Substanzen im Zusammenhang mit Phantomphänomenen, nämlich dem akuten Tinnitus erkannt und vorgeschlagen.

Ein weiterer nicht beanspruchter Gegenstand betrifft ein Verfahren zur Diagnose der Phantomphänomene des akuten Tinnitus bei einem tierischen oder menschlichen Lebewesen, das folgende Schritte aufweist: (a) Bereitstellung einer biologischen Probe des Lebewesens, (b) Bestimmung des Niveaus der Expression von BDNF in der biologischen Probe, (c) Vergleich des Niveaus aus Schritt (b) mit einem Referenzwert aus einem gesunden Lebewesen, und (d) Korrelation eines über dem eines gesunden Lebewesens liegenden Niveaus mit einer positiven Diagnose.

Es kann eine jegliche derartige biologische Probe verwendet werden, die jedoch vorzugsweise aus dem Ohr stammt, wie eine Gewebeprobe, Zellen, bspw. cochleäre Ganglien oder Nervenzellen. Bei der biologischen Probe kann es sich aber auch um eine systemische Blutprobe handeln; Lang et al. (2005), Association of BNDF serum concentrations with central serotonergic activity: Evidence from auditory signal processing, Neuropsychopharmacology 30 (6), Seiten 1148-1153. Dabei ist darauf zu achten, dass es bei einer Gewebeprobenentnahme aus dem Ohr nicht zur Schädigung des Gehörs kommt, wobei solch eine Vorsichtsmaßnahme dann nicht einzuhalten ist, wenn es sich um einen Tinnitus handelt, der sich zentral zu einem nicht mehr intakten Ohr ausbildet. Eine solche Probenentnahme zur Bestimmung des Niveaus der Expression von BDNF kann dann auch gleichzeitig zur Messung der Funktionsfähigkeit der Übertragung und Nutzung zentralerwärts gelegener Nerven genutzt werden, um z.B. die Effizienz der Implantation eines Cochlea-Implantats zu optimieren.

Das nicht beanspruchte Verfahren wird in einem geeigneten biologischen System durchgeführt, wobei übliche Puffer wie Tris-oder HEPES-Puffer Verwendung finden können. Die Bestimmung des Expressionsniveaus in Schritt (b) erfolgt über übliche molekular- oder zellbiologische Methoden, wie ELISA-Technik und Westernblot auf Proteinebene, oder Northernblot auf mRNA-Ebene. Geeignete molekularbiologische Methoden sind bspw. beschrieben in Sambrook, J. und Russel, D. W. (2001), Molecular Cloning - A Laboratory Manual, 3rd Edition, Cold Spring Harbor Laboratory Press, Cold Spring Harbor, New York, der Inhalt dieser Publikation ist durch Inbezugnahme Bestandteil der vorliegenden Beschreibung.

Eine molekularbiologische Diagnose des akuten Tinnitus kann etabliert und bereitgestellt werden.

Zur Behandlung der Phantomphänomene des akuten Tinnitus bei einem menschlichen Lebewesen können folgende Schrittedurchgeführt werden: (a) Bereitstellen eines Arzneimittels, das eine die Blockade oder Inhibition des BDNF-Rezeptors bewirkende oder mit der BDNF-Signaltransduktionskaskade interagierende Substanz sowie einen pharmazeutisch akzeptablen Träger und ggf. weitere Hilfsstoffe und/oder Wirkstoffe aufweist, und (b) Applizieren, ggf. lokal, des Arzneimittels dem Lebewesen, und ggf. (c) Wiederholen der Schritte (a) und (b).

Pharmazeutisch akzeptable Träger und weitere Hilfsstoffe sind im Stand der Technik hinreichend bekannt, vgl. bspw. Kibbe, A. H. (2000), Handbook of Pharmaceutical Excipients, 3rd Edition, American Pharmaceutical Association and Pharmaceutical Press, der Inhalt dieser Publikation ist durch Inbezugnahme Bestandteil der vorliegenden Beschreibung.

Als weitere Wirkstoffe kommen bspw. klassische Tinnitusmittel in Frage.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind.

Die Erfindung wird nun anhand von Ausführungsbeispielen näher erläutert werden.

In den beiliegenden Figuren ist Folgendes dargestellt:
- Fig. 1:: Die Operation und lokale Zufuhr von Salicylat verändert nicht signifikant die Hörschwellen;
- Fig. 2: Akute systemische und lokale Applikation von Salicylat führt nach einer bestimmten Zeit zu einer gesteigerten c-fos-Expression in der Cochlea;
- Fig. 3: *In situ*-Hybridisierungsanalyse von c-fos und BDNF-Exon III und Exon IV-Splice-Varianten in der Cochlea von adulten Ratten nach lokaler und systemischer Applikation von Salicylat;
- Fig. 4: Eine RT-PCR-Analyse zeigt eine dosisabhängige differenzielle Veränderung der Expression von c-fos und BDNF-Exon III- und Exon IV-Expression in der Cochlea nach systemischer und lokaler Applikation von Salicylat;
- Fig. 5: Die lokale Applikation von Salicylat reduziert die Expression von c-fos, BDNF Exon III- und Exon IV in dem auditorischen Cortex;
- Fig. 6: Die durch Salicylat bedingte Hochregulation der BDNF-Expression in cochleären Neuronen wird durch den Typ-Ca⁺⁺-Kanal-Antagonisten Nifedipin inhibiert; und
- Fig. 7: Die durch Salicylat bedingte Hochregulation der BDNF-Expression in cochleären Neuronen wird durch das Benzodiazepin Midazolam inhibiert.

### Ausführungsbeispiel

### MATERIAL UND METHODEN

### Tiere

Für die Experimente wurden weibliche adulte Wistar-Ratten verwendet, die zwischen 280 und 300 Gramm wogen. Die Behandlung und der Umgang mit diesen Tieren erfolgte in Übereinstimmung mit den Institutsrichtlinien der Universität Tübingen, Tierforschungsanstalt.

Die Ratten wurden gemäß dem Verfahren von Guitton, M. J. et al. (2003), Salicylate induces tinnitus through activation of cochlear NMDA receptors, J. Neurosci. 23, Seiten 3944 bis 3952, intraperitoneal anästhesiert, um ein sog. Gelfoam (Gelita Tampon; B. Braun Medical, Melsungen, Deutschland) über das runde Fenster beider Ohren zu platzieren. Das Gelfoam wurde wie angegeben mit Salicylat, das in artifizieller Perilymphlösung (70 mg/ml) verdünnt wurde, oder mit dem entsprechenden Volumen von artifizieller Perilymphe alleine, getränkt. Das Gelfoam wurde auf der Nische des runden Fensters platziert, wie beschrieben; Guitton J. et al. (a.a.O.). Das Salicylat wurde so für 20 Stunden lokal appliziert. Nach dieser Zeit wurden die Tiere getötet und die Cochlea und der auditorische Cortex entfernt.

### Gewebepräparation

Die Tiere wurden mit Kohlenstoffdioxid tiefanästhesiert und anschließend decapiert. Die Cochleae wurden isoliert und wie zuvor beschrieben präpariert; vgl. Knipper, M. et al. (2000), Thyroid hormone deficiency before the onset of hearing causes irreversible damage to peripheral and central auditory systems, J. Neurophysiol. 83, Seiten 3101 bis 3112. Kurz, die Cochleae wurden durch Immersion in 2 % Paraformaldehyd, 125 mM Saccharose in 100 mM phosphatgepufferter Saline (PBS), pH 7,4, für zwei Stunden fixiert, nach der Fixierung für 45 Minuten in "Rapid bone decalcifier (#904687, Eurobio, Fisher-Scientific, 61130 Nidderau, Deutschland) decalcifiziert, anschließend gefolgt von einer Inkubation über Nacht in 25 % Saccharose, 1 mM Proteaseinhibitor (Pefabloc, Roche) in Hanks-gepufferter Saline. Nach der Übernachtinkubation wurden die Cochleae in O.C.T.-Verbindung (Miles Laboratories, Elkhart, Ind., USA) eingebettet. Die Gewebe wurden dann bei 10 µm Dicke für die *in situ-*Hybridisierung kryogeschnitten, gelagert auf SuperFrost*/plus-Objektträger und vor der Verwendung bei -20°C gelagert.

Die auditorischen Cortices wurden gemäß der Beschreibungen identifiziert, die in Paxinos, G. & Watson, C. (1998), The rat brain in stereotaxic coordinates, Academic Press, Inc., gegeben werden. Für RNA-Präparationen wurden die Gewebe unmittelbar in flüssigem Stickstoff eingefroren und vor der Verwendung bei -70°C gelagert.

### Ribosondensynthese und in situ-Hybridisierung

Genomische DNA aus Rattenlebergewebe wurde mittels des Easy-DNA-Kits von Invitrogen gemäß den Angaben des Herstellers isoliert. Eine Polymerasekettenreaktion wurde durchgeführt, um alle vier nicht codierenden 5'-Exons des BDNF-Gens zu amplifizieren.

Um die Exon-3-spezifische Sonde zu amplifizieren, wurde ein Sinn(Sense)-Primer (5' acc cac ttt ccc att cac cg 3') und ein Gegensinn(Antisense)-Primer (5' cct ttt tca gtc act act tg 3') jeweils entsprechend den Nucleotidpositionen 536 bis 555 und 957 bis 976 des genomischen Fragmentes B (Timmusk, T. et al. (1995), Identification of brain-derived neurotrophic factor promoter regions mediating tissue-specific, axotomy-, and neuronal activity-induced expression in transgenic mice, J. Cell. Biol. 128, Seiten 185 bis 199, verwendet. Für die Exon-4-spezifische Sonde wurde ein Sinn-Primer (5' cca atc gaa gct caa ccg aa 3') und ein Gegensinn-Primer (5' tca ggg tcc aca caa agc tc 3') entsprechend den Nucleotidpositionen 1732 bis 1751 und 2059 bis 2078 des genomischen Fragmentes B verwendet (Timmusk, T. et al., a.a.O.). Um die gemeinsame Ribosonde für das codierende Exon-5 zu amplifizieren, wurde ein Sinn-Primer (5' gag gac cag aag gtt cg 3') und ein Gegensinn-Primer (5' ttt atc tgc cgc tgt gac 3') entsprechend der Nucleotidpositionen 309 bis 325 und 534 bis 551 verwendet (Zugangsnummer M61175). Um die c-fos-Sonde zu synthetisieren, wurde ein Sinn-Primer (5' gac ttt tgc gca gat ctg tc 3') und ein Gegensinn-Primer (5' ctg ctc tac ttt gcc cct tc 3') entsprechend den jeweiligen Nucleotidpositionen 276 bis 295 und 508 bis 527 der cDNA verwendet (Zugriffsnummer X06769).

In der PCR-Reaktion wurde die genomische DNA zuerst für vier Minuten bei 94°C denaturiert, gefolgt von 30 Zyklen von einer Minute bei 94°C, einer Minute bei 55°C und einer Minute bei 72°C. Die Verlängerungsreaktion wurde bei 72°C für zehn Minuten durchgeführt. Die amplifizierten Fragmente wurden in einem 1%igen Agarosegel in 1 x TAE-Puffer aufgetrennt. Fragmente, die den erwarteten Längen der genspezifischen Sonden entsprechen, wurden unter Verwendung des QIAquick-Gel-Extraction-Kits von Qiagen extrahiert. Die erwarteten Längen der amplifizierten Fragmente waren 351 Nucleotide (Exon-III), 347 Nucleotide (Exon-IV), 243 Nucleotide (Exon-V) und 252 Nucleotide (c-fos). Diese Fragmente wurden in einen pCR II Topo-Vektor (Invitrogen) kloniert und ihre Nucleotidsequenzen wurden mittels eines automatischen Sequenzierers verifiziert.

Die Plasmide wurden unter Verwendung eines QIAprep Spin Miniprep-Kits von Qiagen isoliert. Um linearisierte Plasmide zur Synthese von Sinn-Ribosonden zu synthetisieren, wurden die Plasmide zuerst mit geeigneten Restriktionsenzymen linearisiert. Ribosonden wurden unter Verwendung von Sp6-, T3- oder T7-RNA-Polymerasen synthetisiert und unter Verwendung von rNTP-Mix markiert, der digoxigeninmarkierte Uridintriphosphate enthält. Sämtliche Restriktionsenzyme, RNA-Polymerasen und digoxigeninmarkierte rNTPs wurden von Roche Diagnostics bezogen. Die *in situ*-Hybridisierung wurde durchgeführt, wie zuvor beschrieben (Wiechers, B. et al. (1999), A changing pattern of brain-derived neurotrophic factor expression correlates with the rearrangement of fibers during cochlea development of rats and mice, J. Neurosci. 19, Seiten 3033 bis 3042. Die Schnitte wurden mit Moviol (Sigma) bedeckt und unter Verwendung eines Olympus AX70-Mikroskops betrachtet.

### Reverse Transkription, semiquantitative PCR

Gesamt-RNA wurde unter Verwendung des Total-RNeasy-Kits von Qiagen sowohl aus der Cochlea als auch aus Hirngewebe isoliert und mit DNase (Ambion) behandelt, um DNA-Kontaminanten zu entfernen. Die RNA wurde anschließend gereinigt und die Konzentrationen mittels spektrometrischer Messungen bestimmt. Die reverse Transkription der Hirngewebe wurde in einem 20 µl Reaktionsvolumen unter Verwendung von 1 µg Gesamt-RNA als Template und SUPERSCRIPT II Rnase H⁻-reverse Transkriptase gemäß dem Protokoll von Invitrogen durchgeführt. Wegen der begrenzten Menge von RNA in den cochleären Geweben wurde unter Verwendung von 50 ng Gesamt-RNA als Template Qiagen Senscript reverse Transkriptase verwendet, wie im Protokoll von Qiagen beschrieben. Für die PCR wurde die Anzahl der Zyklen und die Anlagerungstemperatur optimiert, so dass die Signale, die für BDNF, c-fos und Glyceraldehyd-3-phosphat-Dehydrogenase (GAPDH) erhalten wurden, nicht gesättigt waren.

Die PCR-Primersequenzen für arc waren 5' caa tgt gat cct gca gat tg 3' und 5' tgt tgg cat agg ggc taa ca 3'; für BDNF waren diese 5' ttc gac ccc gcc cgc cgt gg3' und 5' ccc ctt tta atg gtc agt gt 3'; für c-fos waren diese 5' gac ttt tgc gca gat ctg tc 3' und 5' att cct ttc cct tcg gat tc 3'; für GAPDH waren diese 5' tct act gc gtc ttc ac acc a 3' und 5' agg aga caa cct ggt cct cag t 3'. Die PCR wurde in einem 25 µl Endreaktionsvolumen durchgeführt, wobei gleichzeitig beide Primer für GAPDH und die aktivitätabhängigen Gene verwendet wurden. In dieser Duplex-PCR-Reaktion stellt ein Haushaltsgen in der gleichen PCR-Reaktion eine interne Kontrolle dar, so dass die Intensität des aktivitätsabhängigen Gens unzweideutig mit der Kontrolle und den behandelten Proben verglichen werden kann. PCR-readyto-go-Beads von Amershan Pharmacia wurden verwendet, um eine Standardisierung der PCR-Bedingungen zu gewährleisten und Kontaminationen beim Pipettieren zu reduzieren. Letztlich wurden die PCR-Produkte in einem 2%igen Agarosegel analysiert, das mittels einer Ethidium-Bromid-Färbung und Densitometrischer Analyse unter Verwendung eines Alpha-Imagers 2200 von Biometra visualisiert wurde. Die Intensität des amplifizierten aktivitätsabhängigen Gens wurde für jede Reaktion auf den Level des coamplifizierten GAPDH normalisiert. Die Amplifizierungsbedingungen für arc, BDNF, c-fos und GAPDH waren für die anfängliche Denaturierungsphase von 94°C für drei Minuten, 30 Zyklen von jeweils einer Minute Denaturierung (94°C), einer Minute Anlagerung (54°C), 1,5 Minuten Verlängerung (72°C) und einer abschließenden Verlängerungsphase von zehn Minuten bei 72°C. Die PCR-Fragmente wurden kloniert und sequenziert, wie zuvor beschrieben.

### ABR-Screening (ABR = "Auditory brainstem response")

Die Anästhesie der Tiere sowie das ABR-Screening wurde durchgeführt, wie zuvor beschrieben ; vgl. Knipper, M. et al. (2000), a.a.O.; Schimmang, T. et al. (2003), Lack of BDNF and trkB signalling in the postnatal cochlea leads to a spatial reshaping of innervation along the tonotopic axis and hearing loss, Development 130, Seiten 4741 bis 4750.

### ERGEBNISSE

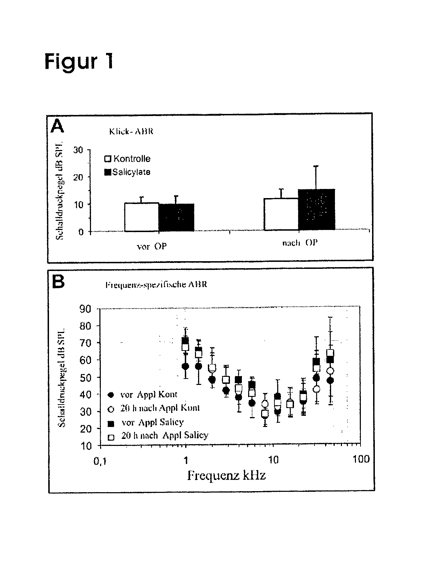

Wie oben beschrieben, wurde weiblichen Ratten etwa gleichen Gewichts Salicylat in die Nische des runden Fensters lokal appliziert. Um jegliche posttraumatischen Auswirkungen auf die Expression der aktivitätsabhängigen Gene in den cochleären Neuronen auszuschließen, wurden ausschließlich Individuen verwendet, die keine Veränderung des Hörvermögens nach Gelfoam-Applizierung zeigten. Wie in Fig. 1 gezeigt, wurden keine signifikanten Veränderungen in dem Klick-abhängigen ABR-Screening vor oder 20 Stunden nach der Operation festgestellt.

Die Schwellen im ABR-Screening waren vor und nach der Operation (OP) weitgehend identisch und es zeigten sich weder bei der Applikation von Salicylat (n = 13; 5 µl; 70mg/kg; Salicylat) noch von artifizieller Perilymphe (n = 9; 5 µl; Kontrolle) wesentliche Unterschiede; vgl. Fig. 1 (A). Ein frequenzspezifisches ABR-Screening vor und 20 Stunden nach lokaler Applikation von Salicylat (n = 5; 5 µl; 70 mg/kg; Salicylat) oder artifizieller Perilymphe (n = 6; 5 µl, Kont) in die Nische des runden Fensters zeigt, dass es zu keinem Hörverlust kommt; vgl. Fig. 1(B).

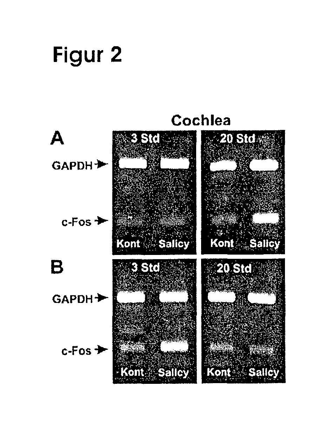

In einem ersten Ansatz zum Herausfinden des geeigneten Zeitpunktes, zu dem entweder lokal oder systemisch appliziertes Salicylat die Cochlea erreicht und die neuronale Erregbarkeit modifiziert, wurde im Cochleagewebe zu verschiedenen Zeitpunkten nach der Salicylatapplizierung über RT-PCR-Analyse die c-fos-Expression untersucht. Dabei wurde festgestellt, dass die Gelfoam-Applikation von Salicylat (5 µl, 70 mg/ml) die c-fos-Expression nicht vor etwa 20 Stunden beeinflusst, möglicherweise auf Grund einer verlangsamten Sekretion der Flüssigkeit aus dem Gelfoam. Eine signifikante Hochregulation von c-fos wurde 20 Stunden, nicht jedoch drei Stunden nach Applizierung festgestellt (Fig. 2A). Hierzu im Gegensatz wurde durch systemische Gabe von Salicylat (350 mg/kg Klörpergewicht) über intraperitoneale Injektion bereits nach drei Stunden eine Hochregulation von c-fos beobachtet ohne signifikante Veränderungen bei längeren Zeiten von bis zu 20 Stunden. Die Expression von GAPDH ist sowohl im Kontrollansatz als auch salicylatbehandelten Ansatz gleich, wodurch demonstriert wird, dass in diesen Experimenten gleiche Mengen von RNA (50 ng) verwendet wurden (Fig. 2B). Um entsprechende akute Wirkungen zu vergleichen, wurden im Folgenden die Effekte von lokal appliziertem Salicylat 20 Stunden und systemisch injiziertem Salicylat drei Stunden nach Applikation untersucht.

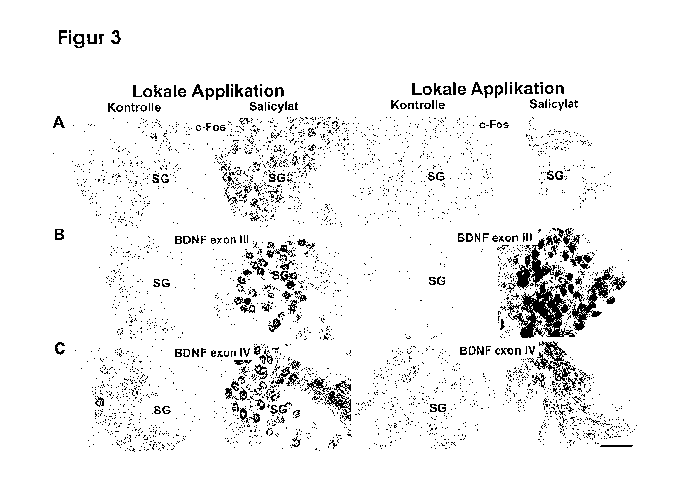

In einem weiteren Schritt wurde der Effekt von lokal und systemisch appliziertem Salicylat auf die Expression bestimmter BNDF-Splice-Varianten in den cochleären Spiralganglien (SG) der Ratte mittels *in situ*-Hybridisierung untersucht. Zum Vergleich wurde die c-fos-Expression beobachtet. Wie exemplarisch in Fig. 3 für drei individuelle Experimente in Duplikaten gezeigt, waren die Hybridisierungssignale für c-fos (Fig. 3A), BDNF-Exon-III (Fig. 3B) und BDNF-Exon IV (Fig. 3C) in der mittleren basalen cochleären Windung im Vergleich zu der Applizierung des gleichen Volumens von artifizieller Perilymphe (Fig. 3, Kontrolle) nach sowohl lokaler (links, 5 µl; 70 mg/ml; lokale Applikation) als auch systemischer (rechts, 350 mg/kg Körpergewicht; systemische Applikation) Gabe von Salicylat signifikant erhöht (dunkle Färbung). Unter Verwendung von Sense-Proben wurden keine Hybridisierungssignale beobachtet (Daten nicht gezeigt). Entlang der tonotopischen Achse zeigte die BDNF-Expression eine charakteristische Abnahme von den basalen/mittelbasalen hin zu den mehr apikalen cochleären Windungen (Schimmang, T. et al. (2003) a.a.O.) und der Salicylat induzierte Anstieg in dem Expressionsmuster beeinflusste primär die mehr basal gelegenen cochleären Windungen.

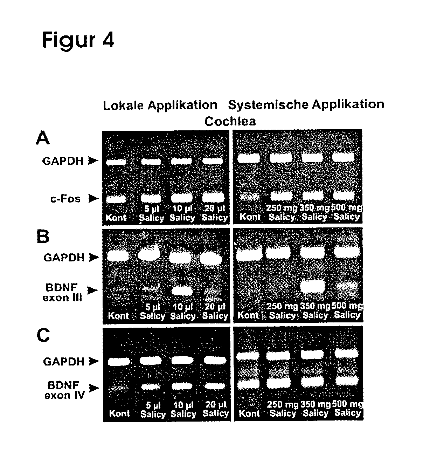

Um mögliche Unterschiede in den Aktivierungsmustern bestimmter BDNF-Transkripte auf verschiedene Anregungsstufen zu untersuchen, wurde der Effekt von verschiedenen Dosierungen von Salicylat analysiert, welche die neuronale Erregbarkeit unterschiedlich beeinflussen könnten; vgl. Kumagai (1992), Effect of intravenous injection of aspirin on the cochlea, Hokkaido Igaku Tasshi 67 (2), Seiten 216 bis 233; Stypulkowski (1990), Mechanisms of salicylate ototoxicity, Hear Res. 46 (1-2), Seiten 113 vis 145. Dazu wurde die Auswirkung von lokal applizierten Gelfoams, getränkt mit verschiedenen Volumina (5, 10, 20 µl) von 70 mg/ml Salicylat und parallel die Auswirkung von verschiedenen Konzentrationen systemisch applizierten Salicylats (250 mg/kg, 350 mg/kg, 500 mg/kg Körpergewicht) untersucht. Unter Verwendung von semiquantitativer RT-PCR-Analyse der Gesamt-RNAs wurden in dem cochleären Gewebe c-fos-, BDNF-Exon-III- und BDNF-Exon-IV-Transkripte untersucht (Fig. 4).

Interessanterweise wurde für beide Applizierungsmethoden ein dosisabhängiger Effekt von Salicylat festgestellt, der im Vergleich zu GADPH zu leichten Veränderungen der Hochregulation der c-fos-Expression führte. Die größten Auswirkungen wurden bei einer Applizierung von 5 µl oder 10 µl Salicylat am runden Fenster bzw. bei einer Injektion von 250 mg/kg oder 350 mg/kg Salicylat festgestellt, wohingegen bei höheren Konzentrationen in beiden Applizierungsmethoden weniger deutliche Auswirkungen festgestellt wurden (Fig. 4A).

Unter Verwendung des gleichen Templates, um sowohl BDNF-Exon-III oder -IV zu amplifizieren, zeigte sich ein deutlicher Unterschied im Aktivierungsmuster der verschiedenen aktivitätsabhängigen Gene.

Unabhängig von der Applizierungsmethode wurde das BDNF-Exon-III mit etwas Verzögerung nach dem BDNF-Exon-IV aktiviert, was bei höheren Konzentrationen von Salicylat in einem Peak der Expression von BDNF-Exon-III (Fig. 4B) resultiert, während sowohl c-fos (Fig. 4A) als auch BDNF-Exon-IV (Fig. 4C) bereits auf geringere Dosen von Salicylat reagieren. Vergleichbare Ergebnisse wurden in drei Experimenten in Duplikaten erhalten, welche die densitometrische Analyse, die in Fig. 4B gezeigt ist, bestätigen, wonach es zu einer signifikanten Erhöhung der Expression des BDNF-Exons-IV (49 ± 12 %, n = 8, p < 0,05) und von c-fos (69 ± 11 %, n = 8, p < 0,05) kommt.

Im auditorischen Cortex wurden die BDNF-Exon-III und -IV-Splice-varianten, das gemeinsame BDNF-Exon-V und c-fos-Transkripte amplifiziert (Daten nicht gezeigt). Es wurden auditorische Cortices untersucht, die aus Tieren erhalten wurden, in denen die Cochlea hinsichtlich der dosisabhängigen Wirkung des Salicylates untersucht wurden, die mRNA wurde isoliert und eine RT-PCR wurde durchgeführt, wie unter Material und Methoden beschrieben.

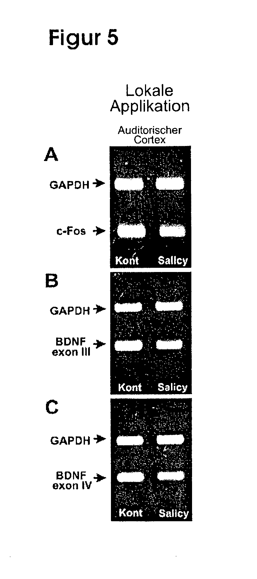

Während lokal oder systemisch appliziertes Salicylat zur Erhöhung der Expression der untersuchten aktivitätsabhängigen Gene in der Cochlea führt (Fig. 4), wurde in drei unabhängigen Experimenten in Duplikaten festgestellt, dass beide Methoden im auditorischen Cortex zu entgegengesetzten Effekten auf die aktivitätsabhängigen Gene führen. Im Gegensatz zur Auswirkung auf die cochleären Neuronen führte die lokale Applikation von sämtlichen Dosierungen von Salicylat zur Reduktion der Expression von c-fos (Fig. 5A, links), von BDNF-Exon-III (Fig. 5B, links) und BDNF-Exon-IV (Fig. 5C, links).

Während in der Cochlea für verschiedene Dosierungen und verschiedene BDNF-Transkripte ein unterschiedliches Aktivierungsmuster festgestellt wurde, war der abnehmende Effekt bei höheren Konzentrationen im auditorischen Cortex nicht so eindeutig (Fig. 5A-C). Mittels densitometrischer Analyse wurde bestätigt, dass es zu einer signifikanten Abnahme der Expression von BNDF-Exon-IV (49 ± 12 %, n = 8, p < 0,05) und c-fos (69 ± 11 %, n = 8, p < 0,05) kommt.

In einem weiteren Experiment haben die Erfinder untersucht, ob das erstmals bei akutem Tinnitus festgestellte Phänomen der erhöhten BDNF-Expression unter Verwendung von Isradipin, einem L-Typ-Ca⁺⁺-Kanal-Antagonisten, der die BDNF-Rezeptor vorgeschaltete Signaltransduktionskaskade beeinflusst, aufgehoben werden kann.

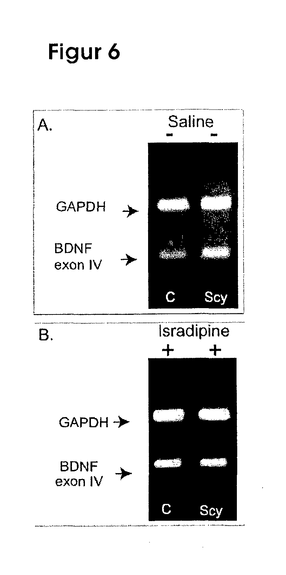

Hierzu wurden 22 Stunden vor der Gewebeentnahme 10 µl 0,9%ige Kochsalzlösung (Fig. 6A, Saline) und 10 µl einer 10 mM Isradipinlösung (Fig. 6B, Isradipine) lokal in die Nische des runden Fensters, d.h. vor die Rundfenstermembran, appliziert. 3 Stunden vor der Gewebeentnahme an der Cochlea wurde identisches Volumen Kochsalzlösung (C) bzw. 350 mg/kg Körpergewicht Salicylat (Scy) systemisch injiziert. Nach der Entnahme des Gewebes wurde die Expression von BDNF in beiden Ansätzen analysiert.

In Fig. 6 ist die Expression des BDNF Exon IV unter vorstehend genannten Bedingungen dargestellt. Wie erwartet bewirkt das Salicylat eine Hochregulation von BDNF in den cochleäre Neuronen (Fig. 6A, rechte Spur), während im identisch durchgeführten Ansatz in der Tiergruppe, in der Isradipin anstelle von Kochsalz appliziert wurde, die Hochregulation von BDNF inhibiert wird.

Das vorstehend beschriebene und in Fig. 6 dargestellte Experiment wurde unter identischen Bedingungen auch für einen weiteren L-Typ-Ca⁺⁺-Kanal-Antagonisten, Nifedipin, durchgeführt. Dabei wurde ebenfalls ein inhibitorischer Effekt auf die nach der Induktion von akutem Tinnitus erhöhte BDNF-Expression in den cochleären Neuronen nachgewiesen, obgleich weniger stark, als mit Isradipin (Daten nicht gezeigt).

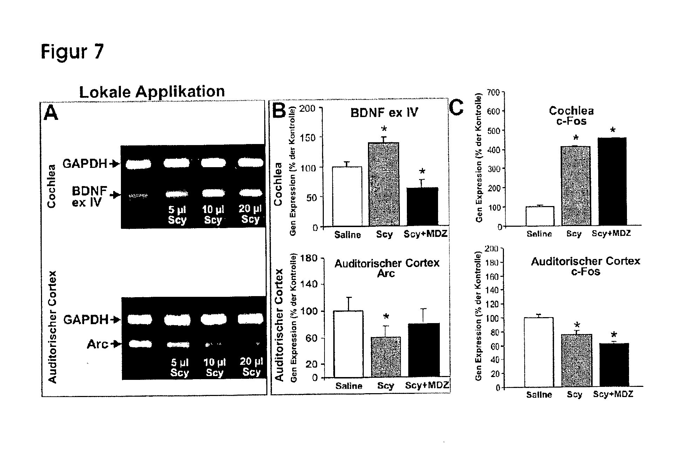

Die Erfinder haben ferner untersucht, ob die mit akutem Tinnitus einhergehende erhöhte BDNF-Expression durch die Verabreichung von GABA-Rezeptor-Agonisten, die mit der vorgeschalteten Signaltransduktionskaskade interagieren, wie Benzodiazepinen, inhibiert werden kann. Wie bereits zuvor beschrieben, wurde weiblichen Ratten steigende Mengen von Salicylat (Scy) in die Nische des runden Fensters lokal appliziert. Dabei zeigte sich, wie auf Grund der zuvor diskutierten Experimente erwartet, eine Zunahme der Expression des BDNF-Exon-IV-Transkriptes in den cochleären Neuronen, und eine Abnahme der Expression des aktivitätsabhängigen Cytoskelettproteins Arc im auditorischen Cortex. Das Ergebnis einer repräsentativen RT-PCR ist in Fig. 7A für n = 3 mit vergleichbarem Ergebnis gezeigt.

In einem weiteren experimentellen Ansatz wurde den Ratten 350 mg/kg Salicylat systemisch appliziert. Wie in Fig. 7A für die lokale Rundfensterapplikation gezeigt, führt auch die systemische Gabe von Salicylat zu einer erhöhten Expression von BDNF-Exon-IV (Fig. 7B, oben) und erwartungsgemäß von c-fos (Fig. 7C, oben) in cochleären Neuronen, während im primären auditorischen Cortex eine Abnahme der Expression von Arc (Fig. 7B, unten) und BDNF-Exon-IV (nicht gezeigt) und gelegentlich von c-fos (Fig. 7C, unten) nachweisbar ist.

Zweieinhalb Stunden nach der Induktion von akutem Tinnitus über die Injektion von 350 mg/kg Salicylat wurde den Tieren systemisch Midazolam (Dormicum, Roche, Grenzach-Wyhlen, Deutschland) (0,5 mg/kg Körpergewicht) verabreicht und die Gen-Expression nach Organentnahme mit Hilfe der RT-PCR untersucht. Dabei stellte sich heraus, dass Midazolam (MDZ) zu einer signifikanten Reduktion des Effektes von Salicylat auf die Expression von BDNF-Exon-IV (n = 7) in den cochleären Neuronen und von Arc im auditorischen Cortex führt (Fig. 7B, rechter Balken, n = 12), die Expression von c-fos jedoch unbeeinflussbar bleibt (Fig. 7C, rechter Balken, n = 7 bis 12). Die statistische Auswertung wurde mit dem "Student's T-test" durchgeführt; * = p < 0,05.

In einem weiteren dem vorstehenden entsprechenden Experiment wurde ein GABA-Rezeptor-Agonist unter ansonsten identischen Bedingungen nicht systemisch sondern lokal in die Nische des Runden Fensters appliziert. Dabei stellte sich heraus, dass die mit akutem Tinnitus verbundene erhöhte BDNF-Exon-IV-Expression in den cochleären Neuronen noch stärker inhibiert wurde und die Auswirkungen auf die Expression von BDNF-Exon-IV im auditiven Cortex deutlich reduziert waren.

Die lokale Applikation eines BDNF-Antagonisten hebt folglich die bei akutem Tinnitus beobachtete pathologische Fehlregulation der BDNF-Expression auf. Die Erfinder konnten deshalb nachweisen, dass es sich bei erfindungsgemäßen BDNF-Antagonisten um Substanzen handelt, die für die Behandlung von akutem Tinnitus grundsätzlich geeignet sind.

Die Erfinder konnten erstmals zeigen, dass mittels Substanzen, die die Blockade oder Inhibition des BDNF-Rezetors bewirken bzw. mit der nachgeschalteten BDNF-Signaltransduktionskaskade interagieren bzw. diese inhibieren, wirksam akuter Tinnitus behandelt werden kann.

## Patentansprüche

1. Substanz, die eine Blockade oder Inhibition des BDNF-Rezeptors (trkB) als trkB-Antagonist oder der diesem Rezeptor nachgeschalteten Signaltransduktionskaskade bewirkt zur Verwendung in der Behandlung der Phantomphänomene des akuten Tinnitus bei einem menschlichen oder tierischen Lebewesen.

2. Substanz zur Verwendung nach Anspruch 1, **dadurch gekennzeichnet, dass** als Substanz ein ein MAP-Kinase-Inhibitor, ein Cam-Kinase-Inhibitor, ein PiB-Kinase-Inhibitor, ein PLC-Inhibitor oder ein Shc-Inhibitor vorgesehen wird.

3. Substanz zur Verwendung nach Anspruch 2, **dadurch gekennzeichnet, dass** als MAP-Kinase-Inhibitor U 0126 oder PD 98058 vorgesehen wird.

4. Substanz zur Verwendung nach einem der Ansprüche 1 bis 3, **dadurch gekennzeichnet, dass** die Substanz lokal am oder im Ohr appliziert wird.

5. Substanz zur Verwendung nach Anspruch 4, **dadurch gekennzeichnet, dass** die lokale Applikation durch die Anwendung eines bioabbaubaren Hydrogels erfolgt, das als Trägermatrix für die Substanz dient

6. Substanz zur Verwendung in der therapeutischen oder prophylaktischen Behandlung der Phantomphänomene des akuten Tinnitus bei einem menschlichen oder tierischen Lebewesen, ausgewählt aus der Gruppe bestehend aus MAP-Kinase-Inhibitoren, insbesondere U 0126 oder PD 98058, PLC-Inhibitoren, PIB-Kinase-Inhibitoren, Shc-Inhibitoren und Cam-Kinase-Inhibitoren.

## Claims

1. Compound that induces a blockade or inhibition of the BDNF-receptor (trkB) as trkB-antagonist or of its downstream signal transduction cascade for use in the treatment of phantom phenomena of acute tinnitus of a human or animal being.

2. Compound for use according to claim 1, wherein the compound to be used is a MAP-Kinase-inhibitor, a Cam-Kinase-inhibitor, a Pi3-Kinase-inhibitor, a PLC-inhibitor or an Shc-inhibitor.

3. Compound for use according to claim 2, wherein the compound to be used is MAP-kinase-inhibitor U 0126 or PD 98058.

4. Compound for use according to any of claims 1 to 3, wherein the compound is applied locally on or at the ear.

5. Compound for use according to claim 4, wherein the local application is effected by the use of a biodegradable hydrogel, which serves as carrier matrix for the compound.

6. Compound for use in the therapeutic or prophylactic treatment of phantom phenomena of acute tinnitus of a human or animal being, selected from the group consisting of: MAP-Kinase-inhibitors, preferably U 0126 or PD 98058, PLC-inhibitors, Pi3-Kinase-inhibitors, Shc-inhibitors and Cam-Kinase-inhibitors.

## Revendications

1. Substance qui provoque un blocage ou une inhibition du récepteur du BDNF (trkB) en tant qu'antagoniste du trkB, ou de la cascade de transductions de signal en aval de ce récepteur, pour utilisation dans le traitement des phénomènes fantômes de l'acouphène aiguë chez un être vivant humain ou animal.

2. Substance pour l'utilisation selon la revendication 1, **caractérisée en ce qu'**on prévoit en tant que substance un inhibiteur de la MAP-kinase, un inhibiteur de la Cam-kinase, un inhibiteur de la Pi3-kinase, un inhibiteur de la PLC ou un inhibiteur de la Shc.

3. Substance pour l'utilisation selon la revendication 2, **caractérisée en ce qu'**on prévoit en tant qu'inhibiteur de la MAP-kinase l'U 0126 ou le PD 98058.

4. Substance pour l'utilisation selon l'une des revendications 1 à 3, **caractérisée en ce que** la substance est appliquée localement sur ou dans l'oreille.

5. Substance pour l'utilisation selon la revendication 4, **caractérisée en ce que** l'application locale a lieu par utilisation d'un hydrogel biodégradable, qui sert de matrice formant support pour la substance.

6. Substance pour l'utilisation dans le traitement thérapeutique ou prophylactique des phénomènes fantômes de l'acouphène aiguë chez un être vivant humain ou animal, choisie dans le groupe consistant en les inhibiteurs de la MAP, en particulier l'U 0126 ou le PD 98058, les inhibiteurs de la PLC, les inhibiteurs de la Pi3-kinase, les inhibiteurs de la Shc et les inhibiteurs de la Cam-kinase.
